# EUROPÄISCHE PATENTANMELDUNG

(11) **EP 4 578 438 A2**
(43) Veröffentlichungstag der Anmeldung: **02.07.2025**
(21) Anmeldenummer: 24217732.7
(22) Anmeldetag: 05.12.2024
(51) Int. Cl.: A61K 6/17, A61K 6/77, A61K 6/887, A61K 6/90

(54) **ABFORMMATERIALIEN MIT VERBESSERTEN RHEOLOGISCHEN EIGENSCHAFTEN**

(30) Priorität: 19.12.2023 DE 102023135715
(71) Anmelder: Kulzer GmbH, 63450 Hanau (DE)
(72) Erfinder: Geißler, Tobias, 63584 Gründau (DE); Grundler, Andreas, 35516 Münzenberg (DE); Kapala, Natalie, 65933 Frankfurt am Main (DE); Hamburg, Roman, 35410 Hungen (DE); Deutzmann, Peter, 41569 Rommerskirchen (DE)
(74) Vertreter: Pelster Behrends Patentanwälte PartG mbB

(57) **Zusammenfassung**

Die Erfindung betrifft ein Mehrkomponentensystem zur Herstellung einer dentalen Abformasse, umfassend in zwei oder mehr separaten Komponenten: i) ein oder mehrere vernetzbare (Co-)Polymere, ii) eine oder mehrere Bestandteile eines Vernetzersystems zur Vernetzung der vernetzbaren (Co-)Polymere, und iii) einen oder mehrere Füllstoffe, wobei das Mehrkomponentensystem zumindest einen plättchenförmigen Füllstoff umfasst, wobei der plättchenförmige Füllstoff einen ersten Formfaktor F1 = Dₒᵣₜₕₒ/Dₘₐₓ im Bereich von 0,3 bis 1,0 und einen zweiten Formfaktor F2 = Dₘₐₓ/H von 3 oder mehr aufweist, wobei Dₘₐₓ der maximale Plättchen-Durchmesser in der Plättchen-Ebene ist, wobei Dₒᵣₜₕₒ der größte Plättchen-Durchmesser orthogonal zu Dₘₐₓ in der Plättchen-Ebene ist, und wobei H die mittlere Plättchen-Dicke orthogonal zur Plättchen-Ebene ist.

## Beschreibung

Die Erfindung betrifft ein Mehrkomponentensystem zur Herstellung einer dentalen Abformasse, eine daraus hergestellte dentale Abformmasse und die Verwendung eines plättchenförmigen Füllstoffs als Füllstoff in dentalen Abformmassen. Offenbart wird zudem die Verwendung eines entsprechenden Mehrkomponentensystems zur Herstellung einer dentalen Abformmasse.

Techniken der Zahn- und Kieferabformung spielen auch in der modernen Zahnheilkunde beziehungsweise im Alltag von Zahntechnikern unverändert eine wichtige Rolle. Hierbei werden beispielsweise die relevanten Strukturen im Kiefer des Patienten unter Einsatz von sogenannten Abformmassen abgebildet. Diese Abformmassen werden dabei zumeist als Mehrkomponentensysteme von zwei oder mehr Komponenten bereitgestellt, welche vom Anwender erst unmittelbar vor dem Einsatz vermischt werden, um die tatsächliche Abformmasse zu erhalten. Die Mehrkomponentensysteme sind dabei so ausgelegt, dass die Vermischung der einzelnen Komponenten zu einer Aushärtung der Abformmasse führt, so dass im Ergebnis eine vergleichsweise feste und harte Negativform der abgebildeten Strukturen erhalten wird, welche im Zuge nachgelagerter Verarbeitungsschritte verwendet werden kann, beispielsweise bei der Herstellung dentaler Bauteile.

Informationen zum allgemeinen technischen Hintergrund sind beispielsweise in der EP 0613926 B1, der EP 1946740 A2, der US 2005/0239958 A1, der EP 2219585 B1 oder der US 6599974 B1 offenbart.

An entsprechende Mehrkomponentensysteme beziehungsweise die daraus herstellbaren dentalen Abformmassen werden heutzutage hohe Anforderungen gestellt. Viele der angestrebten Eigenschaften solcher Mehrkomponentensysteme betreffen die Verarbeitbarkeit der einzelnen Komponenten bzw. der dentalen Abformmasse. Für viele der relevanten Aspekte bestehen dabei Zielkonflikte von konkurrierenden Eigenschaften, welche nicht unabhängig voneinander optimiert werden können und bei denen ein Bedürfnis danach besteht, eine möglichst vorteilhafte Lösung des Zielkonflikts zu finden. Ein Beispiel für einen solchen Zielkonflikt ist dabei beispielsweise die Konkurrenz zwischen einer hinreichend langen Verarbeitungszeit der gemischten Abformmasse, welche dem Anwender genug Zeit gibt, den gewünschten Abdruck zu nehmen, und einer vorteilhaften Aushärtungskinetik, welche die Behandlungsdauer für den Patienten möglichst minimiert.

Ein wichtiger Zielkonflikt besteht auch zwischen den rheologischen Eigenschaften, insbesondere der Viskosität und dem Fließverhalten, der Komponenten bzw. der noch nicht vollständig ausgehärteten Abformmasse auf der einen Seite und den mechanischen Eigenschaften der ausgehärteten dentalen Abformmasse auf der anderen Seite. Die einzelnen Komponenten des Mehrkomponentensystems sollen nämlich eine vorteilhaft niedrige Viskosität aufweisen, so dass sich diese beispielsweise leicht aus den entsprechenden Gebinden bereitstellen und in effizienter Weise miteinander vermischen lassen.

Auch an die aus den Komponenten erhaltene dentale Abformmasse werden während der Verarbeitungszeit hohe Anforderungen an die Viskosität und das Fließverhalten gestellt. Hierbei ist es wünschenswert, dass die Abformmassen bei der Applikation hinreichend standfest sind, so dass sie im Mund des Patienten nicht auslaufen, unter geringem Druck jedoch fließen und alle gewünschten Details, z.B. der präparierten Zähne und des Sulkus, genau abformen können. Ein vorteilhaftes Fließverhalten ist insbesondere beim Einsetzen des Löffels in den Patientenmund oder beim Umspritzen von Zähnen oder Zahnstümpfen initial sehr wichtig, damit detailgenaue Abdrücke genommen werden können. Diese Eigenschaften spielen insbesondere bei einseitigen Abformtechniken wie der Monophasenabformtechnik eine große Rolle. Ein gutes Fließverhalten kann auch bei als Bissregistriermaterialien eingesetzten Abformmassen vorteilhaft sein, da diese idealerweise nach der Applikation standfest auf dem Zahnbogen verbleiben, gleichzeitig aber unter geringem Gegendruck das Zusammenbeißen von Ober- und Unterkiefer ermöglichen müssen, ohne dabei eine Bissverschiebung zu bewirken.

Den gewünschten rheologischen Eigenschaften stehen in vielen Punkten die Anforderungen entgegen, welche an die mechanischen Eigenschaften der ausgehärteten dentalen Abformmaterialien gestellt werden. Für diese ist nämlich regelmäßig eine relativ hohe Endhärte gewünscht, wobei die erhaltenen elastomeren Materialien darüber hinaus auch eine vorteilhafte Reißdehnung aufweisen sollen, was insbesondere bei Silikon-basierten Abformmaterialien in vielen Fällen eine besondere Herausforderung darstellt, da in diesen eine hohe Endhärte häufig mit einer verringerten Reißdehnung einhergeht. Die Realisierung dieser gewünschten Eigenschaften erfordert regelmäßig den Einsatz hoher Füllstoffgehalte. Solche hohen Füllstoffgehalte haben jedoch zumeist einen negativen Einfluss auf die rheologischen Eigenschaften der Komponenten bzw. der noch nicht vollständig ausgehärteten Abformmasse.

Die primäre Aufgabe der vorliegenden Erfindung war es, die Nachteile des Standes der Technik auszuräumen oder zumindest abzuschwächen.

Insbesondere war es eine Aufgabe der vorliegenden Erfindung, ein Mehrkomponentensystem zur Herstellung einer dentalen Abformasse anzugeben, welches den Zielkonflikt zwischen den rheologischen Eigenschaften der Komponenten bzw. der noch nicht vollständig ausgehärteten Abformmasse auf der einen Seite und den mechanischen Eigenschaften auf der anderen Seite in vorteilhafter Weise löst.

Insoweit war es eine Aufgabe der vorliegenden Erfindung, dass das anzugebende Mehrkomponentensystem es ermöglichen sollte, die einzelnen Komponenten mit einer vorteilhaft niedrigen Viskosität auszuführen, so dass sich diese insbesondere mit einer niedrigen Auspresskraft aus einer Kartusche auspressen und leicht miteinander vermischen lassen.

Zudem war es eine Aufgabe der vorliegenden Erfindung, dass das anzugebende Mehrkomponentensystem durch Mischen der Komponenten in ein dentales Abformmaterial überführbar sein sollte, welches ausreichend standfest ist, gleichzeitig aber über ein vorteilhaftes Fließverhalten verfügt, mit dem sich insbesondere präzise und detailreiche Abformungen erhalten lassen, insbesondere auch in schwer zugänglichen Bereichen.

Darüber hinaus war es eine Aufgabe der vorliegenden Erfindung, dass das anzugebende Mehrkomponentensystem durch Mischen der Komponenten in ein dentales Abformmaterial überführbar sein sollte, welches nach Aushärtung vorteilhafte mechanische Eigenschaften aufweisen sollte, insbesondere eine hohe Endhärte und eine vorteilhafte Reißdehnung. Insoweit war es wünschenswert, dass die anzugebende Lösung für eine breite Spanne an Füllstoffgehalten anwendbar sein sollte, insbesondere auch für hohe Gesamtfüllstoffgehalte.

Vor diesem Hintergrund war es eine Aufgabe der vorliegenden Erfindung, dass die anzugebende Lösung möglichst keinen nachteiligen Einfluss auf die Aushärtekinetik der dentalen Abformmaterialien haben sollte. Zudem war es wünschenswert, dass die anzugebende Lösung möglichst keine zusätzlichen Anforderungen an die zur Herstellung der anzugebenden Mehrkomponentensysteme notwenigen Vorrichtungen und Verfahren stellen sollte.

Es war eine wünschenswerte Maßgabe, dass die anzugebenden Mehrkomponentensysteme in möglichst zeit- und kosteneffizienter Art und Weise herstellbar sein sollten und dass die gefundene Lösung ohne den Einsatz potentiell gesundheits- und/oder umweltschädlicher Materialien auskommen sollte.

Es war eine weitere Aufgabe der vorliegenden Erfindung, eine aus dem anzugebenden Mehrkomponentensystem hergestellte dentale Abformmasse bereitzustellen, welche über ausgezeichnete Verarbeitungseigenschaften bei gleichzeitig vorteilhaften mechanischen Eigenschaften verfügt. Insoweit war es eine sekundäre Aufgabe der vorliegenden Erfindung, eine Verwendung des anzugebenden Mehrkomponentensystems zur Herstellung einer dentalen Abformmasse anzugeben.

Zudem war es eine Aufgabe der vorliegenden Erfindung eine Verwendung für einen plättchenförmigen Füllstoff in dentalen Abformmassen anzugeben.

Die Erfinder der vorliegenden Erfindung haben nunmehr gefunden, dass sich die vorstehend beschriebenen Aufgaben überraschenderweise lösen lassen, wenn in einem Mehrkomponentensystem zur Herstellung einer dentalen Abformasse als Füllstoff zumindest ein plättchenförmiger Füllstoff eingesetzt wird, wie es in den Ansprüchen definiert ist.

Überraschenderweise lassen sich durch den Einsatz von solchen plättchenförmigen Füllstoffen Mehrkomponentensysteme zur Herstellung dentaler Abformasse erhalten, welche den Zielkonflikt zwischen den rheologischen Eigenschaften der Komponenten bzw. der noch nicht vollständig ausgehärteten Abformmasse auf der einen Seite und den mechanischen Eigenschaften auf der anderen Seite in vorteilhafter Weise löst, wobei gute Verarbeitungseigenschaften für die Verwendung der dentalen Abformmasse als auch für die Weiterverarbeitung der damit herstellbaren Abdrücke erzielt werden können. In vorteilhafter Weise sind dabei auch hohe Füllstoffgehalte möglich. Zudem lassen sich durch den Einsatz entsprechender plättchenförmiger Füllstoffe insbesondere auch in silikon-basierten Abformmassen trotz einer hohen Endhärte günstige Reißeigenschaften erzielen. Darüber hinaus hat der Einsatz solcher plättchenförmigen Füllstoffe in vorteilhafter Weise keinen bzw. keinen spürbaren negativen Einfluss auf die Aushärtekinetik der dentalen Abformmaterialien.

Die vorstehend genannten Aufgaben werden somit durch den Gegenstand der Erfindung gelöst, wie er in den Ansprüchen definiert ist. Bevorzugte erfindungsgemäße Ausgestaltungen ergeben sich aus den Unteransprüchen und den nachfolgenden Ausführungen.

Solche Ausführungsformen, die nachfolgend als bevorzugt bezeichnet sind, werden in besonders bevorzugten Ausführungsformen mit Merkmalen anderer als bevorzugt bezeichneter Ausführungsformen kombiniert. Ganz besonders bevorzugt sind somit Kombinationen von zwei oder mehr der nachfolgend als besonders bevorzugt bezeichneten Ausführungsformen. Ebenfalls bevorzugt sind Ausführungsformen, in denen ein in irgendeinem Ausmaß als bevorzugt bezeichnetes Merkmal einer Ausführungsform mit einem oder mehreren weiteren Merkmalen anderer Ausführungsformen kombiniert wird, die in irgendeinem Ausmaß als bevorzugt bezeichnet werden. Merkmale bevorzugter dentaler Abformmaterialien und Verwendungen ergeben sich aus den Merkmalen bevorzugter Mehrkomponentensysteme.

Insoweit nachfolgend für ein Element, beispielsweise für die plättchenförmigen Füllstoffe oder die vernetzbaren (Co-)Polymere, sowohl spezifische Mengen bzw. Anteile dieses Elementes als auch bevorzugte Ausgestaltungen des Elementes offenbart werden, sind insbesondere auch die spezifischen Mengen bzw. Anteile der bevorzugt ausgestalteten Elemente offenbart. Zudem ist offenbart, dass bei den entsprechenden spezifischen Gesamtmengen bzw. Gesamtanteilen der Elemente zumindest ein Teil der Elemente bevorzugt ausgestaltet sein kann und insbesondere auch, dass bevorzugt ausgestaltete Elemente innerhalb der spezifischen Gesamtmengen oder Gesamtanteile wiederum in den spezifischen Mengen bzw. Anteilen vorliegen können.

Die Erfindung betrifft ein Mehrkomponentensystem zur Herstellung einer dentalen Abformasse, umfassend in zwei oder mehr separaten Komponenten:
i) ein oder mehrere vernetzbare (Co-)Polymere,
ii) einen oder mehrere Bestandteile eines Vernetzersystems zur Vernetzung der vernetzbaren (Co-)Polymere, und
iii) einen oder mehrere Füllstoffe,

wobei das Mehrkomponentensystem zumindest einen plättchenförmigen Füllstoff umfasst,
wobei der plättchenförmige Füllstoff einen ersten Formfaktor F1 = Dₒᵣₜₕₒ/Dₘₐₓ im Bereich von 0,3 bis 1,0 und einen zweiten Formfaktor F2 = Dₘₐₓ/H von 3 oder mehr aufweist,
wobei Dₘₐₓ der maximale Plättchen-Durchmesser in der Plättchen-Ebene ist, wobei Dₒᵣₜₕₒ der größte Plättchen-Durchmesser orthogonal zu Dₘₐₓ in der Plättchen-Ebene ist, und wobei H die mittlere Plättchen-Dicke orthogonal zur Plättchen-Ebene ist.

Dentale Abformmassen und die zu ihrer Herstellung verwendeten Mehrkomponentensysteme als solche sind dem Fachmann aus dem Stand der Technik prinzipiell umfassend bekannt.

In Übereinstimmung mit dem fachmännischen Verständnis und dem üblichen Vorgehen im Bereich der Technik werden die erfindungsgemäßen Mehrkomponentensysteme dabei über die im Mehrkomponentensystem enthaltenen Bestandteile definiert, welche in zwei oder mehr separaten Komponenten des Mehrkomponentensystems vorgelegt werden, beispielsweise in unterschiedlichen Gebinden. Dabei können einzelne Bestandteile des Mehrkomponentensystems auch in zwei oder mehr, bzw. sämtlichen, der Komponenten vorliegen. Die Herstellung der dentalen Abformmasse aus den Komponenten des Mehrkomponentensystems erfolgt regelmäßig durch Mischen der Komponenten, was beispielsweise durch eine geeignete Mischvorrichtung befördert oder bedingt werden kann, wobei geeignete Mischvorrichtungen, welche beispielsweise als Mischspritzen ausgeführt sein können, von zahlreichen Herstellern kommerziell verfügbar sind. Bevorzugt ist wegen einer einfachen Handhabung von lediglich zwei Komponenten ein erfindungsgemäßes Mehrkomponentensystem, wobei das Mehrkomponentensystem ein Zweikomponentensystem ist, welches die Bestandteile i), ii) und iii) aufgeteilt in zwei separate Komponenten umfasst. Für die Praxis besonders relevant ist ein erfindungsgemäßes Mehrkomponentensystem, wobei die separaten Komponenten des Mehrkomponentensystems in verschiedenen Gebinden, bevorzugt in separaten Tuben oder in separaten Kammern einer Kartusche zum Einsatz in eine Mischvorrichtung, bevorzugt in separaten Kammern einer Kartusche zum Einsatz in einer Mischspritze vorliegen.

Diese vorstehend definierten Bestandteile des Mehrkomponentensystems werden in Übereinstimmung mit dem fachmännischen Verständnis jeweils als "ein oder mehrere" eingesetzt. Die Bezeichnung "ein oder mehrere" bezieht sich dabei in branchenüblicher Weise auf die chemische Natur der entsprechenden Verbindungen und nicht auf deren Stoffmenge. Beispielsweise kann das Mehrkomponentensystem lediglich eine Art von vernetzbaren (Co-)Polymeren umfassen, was bedeuten würde, dass das Mehrkomponentensystem eine Vielzahl der entsprechenden Moleküle umfasst.

Insoweit im Rahmen der Erfindung Massenanteile angegeben sind, werden dabei in branchenüblicher Weise die kombinierte Massenanteile der einen oder mehrerer Komponenten angegeben, wodurch ausgedrückt wird, dass der Massenanteil der entsprechend ausgebildeten Komponenten zusammengenommen die entsprechenden Kriterien erfüllt, wobei in Abwesenheit anderer Angaben jeweils die Gesamtmasse des Mehrkomponentensystems das Bezugssystem bildet.

Das erfindungsgemäße Mehrkomponentensystem umfasst in zumindest einer Komponente, bevorzugt in zwei oder mehr der Komponenten, besonders bevorzugt in sämtlichen Komponenten ein oder mehrere vernetzbare (Co-)Polymere. Der Ausdruck "(Co-)Polymere" bringt dabei zum Ausdruck, dass es sich um höhermolekulare Verbindungen handelt, welche aus einer Vielzahl von Monomereinheiten herstellbar sind. In Übereinstimmung mit dem fachmännischen Verständnis sind die Grenzen zwischen Polymeren und Oligomeren nicht scharf definiert. Im Rahmen der vorliegenden Erfindung umfasst der Ausdruck "(Co-)Polymere" aus Gründen einer prägnanten Bezeichnung auch (Co-)Oligomere, wobei der Einsatz von (Co-)Polymeren im engeren Sinne bevorzugt ist.

Die erfindungsgemäß einzusetzenden (Co-)Polymere sind vernetzbar. In Übereinstimmung mit dem fachmännischen Verständnis bedeutet dies, dass sie über eine oder mehrere chemische Funktionalitäten verfügen, über welche eine Vernetzung der (Co-)Polymere möglich ist. Aus dem Stand der Technik ist eine sehr große Zahl an unterschiedlichen Grundsystemen für dentale Abformmassen bekannt, welche auf unterschiedliche vernetzbare (Co-)Polymere setzen. Die Art des Vernetzungsmechanismus der dafür benötigten chemischen Verbindungen hängt dabei von der chemischen Natur der (Co-)Polymere ab, wobei im Stand der Technik eine Vielzahl an unterschiedlichen Vernetzungskonzepten zum Einsatz kommt. Funktional ist diesen sehr unterschiedlichen Kombinationen aus vernetzbaren (Co-)Polymeren und Vernetzungskonzepten dabei jedoch stets gemein, dass die eingesetzten vernetzbaren (Co-)Polymere unter Ausnutzung der jeweiligen chemischen Funktionalitäten durch ein geeignetes Vernetzungssystem vernetzt werden. Auch wenn beispielsweise auch radikalische Mechanismen denkbar sind, ist ein erfindungsgemäßes Mehrkomponentensystem bevorzugt, wobei das Mehrkomponentensystem ein Mehrkomponentensystem zur Herstellung einer dentalen Abformasse mittels Additionsvernetzung oder Kondensationsvernetzung ist, bevorzugt durch Additionsvernetzung.

Die vorliegende Erfindung betrifft in Übereinstimmung mit dem fachmännischen Verständnis insbesondere eine Innovation hinsichtlich der Füllstoffsysteme für dentale Abformmassen. Insoweit haben die Erfinder gefunden, dass sich die vorteilhaften Effekte der vorliegenden Erfindung im Wesentlichen mit sämtlichen Grundsystemen aus (Co-)Polymer und entsprechender Vernetzungschemie realisieren lassen, wodurch zu vermuten steht, dass die vorteilhaften Effekte insbesondere auf physikalischen bzw. mechanischen Wechselwirkungen basieren und die chemische Wechselwirkung mit den Füllstoffen eine geringere Rolle spielt. Diese breite Anwendbarkeit der erfindungsgemäßen Lehre kann aus sich heraus bereits als Vorteil gesehen werden. Besonders günstig ist es insoweit jedoch auch, dass in Folge dieser Feststellung die Notwendigkeit entfällt, die große Zahl an möglichen Kombinationen aus (Co-)Polymeren und den darauf abgestimmten Vernetzungssystemen einzeln zu definieren. Vielmehr kann der Fachmann bei der Umsetzung der Erfindung auf die aus dem Stand der Technik bekannten Grundsysteme für dentale Abformmassen zurückgreifen und diese durch den Einsatz der spezifischen plättchenförmigen Füllstoffe optimieren. Im Lichte dieses Befundes umfasst das erfindungsgemäße Mehrkomponentensystem eine oder mehrere Komponenten, welche beim späteren Vermischen der Vernetzung der (Co-)Polymere dienen und die im Rahmen der vorliegenden Erfindung als Vernetzersystem zusammengefasst werden. Das Vernetzersystem umfasst dabei prinzipiell die Bestandteile, welche aus dem Stand der Technik für die Vernetzung der entsprechenden (Co-)Polymere bekannt sind.

Die Vernetzung kann beispielsweise als Homo-Vernetzung zwischen den (Co-)Polymeren erfolgen, beispielsweise indem eine inhärente Reaktivität durch den Einsatz eines geeigneten Aktivators und/oder Katalysators ausgenutzt wird oder indem mit einem Initiator beispielsweise eine radikalische Polymerisation initiiert wird. Zusätzlich oder alternativ ist es auch möglich, dass eine Hetero-Polymerisation erfolgt, bei dem die (Co-)Polymere zumindest teilweise über weitere Verbindungen, sogenannte Vernetzer, verknüpft werden, wobei die Reaktivität zwischen (Co-)Polymeren und Vernetzer-Verbindungen bei Bedarf wiederum durch Aktivatoren, Katalysatoren und/oder Initiatoren befördert oder bewirkt werden kann. In Abhängigkeit von dem zugrundeliegenden Polymersystem bevorzugt ist entsprechend ein erfindungsgemäßes Mehrkomponentensystem, wobei das Mehrkomponentensystem als Bestandteile des Vernetzersystems umfasst: ii.a) eine oder mehrere Vernetzer-Verbindungen zur kovalenten Vernetzung der vernetzbaren (Co-)Polymere, und/oder ii.b) eine oder mehrere Katalysator-Verbindungen, und/oder ii.c) eine oder mehrere Aktivator-Verbindungen, und/oder iv.d) eine oder mehrere Initiator-Verbindungen, insbesondere Photoinitiatoren.

Wegen der großen Vielfalt an möglichen Grundsystemen, welche mit der vorliegenden Erfindung kompatibel sind, ist es sehr schwer, bevorzugte Massenanteile für die Komponenten anzugeben. Für viele Anwendungen relevant ist nach Einschätzung der Erfinder hinsichtlich der Vernetzer-Verbindungen jedoch ein erfindungsgemäßes Mehrkomponentensystem, wobei der kombinierte Massenanteil der einen oder der mehreren Vernetzer-Verbindungen im Bereich von 0,25 bis 20 %, bevorzugt im Bereich von 1 bis 15 %, besonders bevorzugt im Bereich von 2 bis 10 %, liegt, bezogen auf die Gesamtmasse des Mehrkomponentensystems. Typisch ist beim Einsatz von Katalysatoren und ähnlichen Verbindungen hingegen zusätzlich oder alternativ ein erfindungsgemäßes Mehrkomponentensystem, wobei der kombinierte Massenanteil der einen oder der mehreren Katalysator-Verbindungen und/oder der Aktivator-Verbindungen und/oder/der Initiator-Verbindungen im Bereich von 0,01 bis 0,5 %, bevorzugt im Bereich von 0,02 bis 0,1 % liegt, bezogen auf die Gesamtmasse des Mehrkomponentensystems.

Der Fachmann versteht, dass das Vernetzersystem hinsichtlich seiner Verteilung auf die Komponenten des Mehrkomponentensystems der Maßgabe unterliegt, dass das Vernetzersystem vor dem Mischen der Komponenten nicht - beziehungsweise zumindest nicht im nennenswerten Umfang - zu einer Vernetzung und damit einer Aushärtung der Komponenten führen sollte. Entsprechend ist es für im Wesentlichen alle Ausführungsformen bevorzugt, dass keine der Komponenten des Mehrkomponentensystems sowohl die vernetzbaren (Co-)Polymere als auch sämtliche Bestandteile des Vernetzersystems umfasst.

Zudem ist es möglich, dass das Vernetzersystem verschiedene Bestandteile umfasst, welche eine Vernetzung der (Co-)Polymere über unterschiedliche Vernetzungsmechanismen realisieren kann. Hierdurch ist es in vorteilhafter Weise möglich, eine mehrstufige Vernetzungskinetik zu realisieren und das Aushärtungsverhalten gezielt an die jeweiligen Anwendungserfordernisse anzupassen.

Eine besonders wichtige Gruppe an (co-)polymeren Grundsystemen für Abformmassen sind die sogenannten Polyether-Abformmassen, in denen Polyether, welche beispielsweise mit Aziridino-Gruppen funktionalisiert sein können, eingesetzt werden und welche beispielsweise unter Einsatz einer Säure oder eines anderen kationischen Initiators polymerisiert werden können. Entsprechende Polyether-Abformmassen sind beispielsweise aus der EP 0613926 B1, der EP 1946740 A2 oder der EP 2219585 B1 bekannt. Entsprechende Polyether-Abformmassen werden prinzipiell hinsichtlich vieler relevanter Eigenschaften als vorteilhaft empfunden, insbesondere weil diese Polyether-Abformmassen zumeist eine hohe Endhärte bei guter bis sehr guter Reißdehnung aufweisen. In Kombination mit dem erfindungsgemäß vorgesehenen Einsatz der spezifischen plättchenförmigen Füllstoffe ergeben sich dadurch besonders leistungsstarke Abformmassen. Bevorzugt ist für viele Anwendungen entsprechend ein erfindungsgemäßes Mehrkomponentensystem, wobei das eine oder die mehreren (Co-)Polymere ausgewählt sind aus der Gruppe bestehend aus funktionalisierten Polyethern, bevorzugt Polyethern mit Aziridino-Gruppen, wobei das Vernetzersystem bevorzugt zumindest eine Säure umfasst.

Eine andere Klasse von Grundsystemen, die im Bereich der Technik eine hohe Relevanz aufweisen, sind die sogenannten Silikon-Abformmassen, wie sie beispielsweise in der US 2005/0239985 A1 offenbart werden. Diese prinzipiell bekannten und für viele Anwendungen geeigneten Silikon-Abformmassen werden insbesondere im Vergleich zu Polyether-Abformmassen jedoch teilweise als nachteilig empfunden, da sich in diesen die angestrebte Kombination aus einer hohen Endhärte mit guten bis sehr guten Reißdehnungen häufig nicht oder zumindest nicht leicht realisieren lässt. Die Erfinder haben insoweit gefunden, dass sich bei Adaption der Erfindung auf Silikon-Abformmassen überraschenderweise nicht nur die viskosimetrischen Eigenschaften und das Fließverhalten verbessern lassen, sondern dass sich darüber hinaus durch die Wahl des Füllstoffs auch eine Verbesserung in dem für Silikon-Abformmassen beobachteten Zielkonflikt aus Härte und Reißdehnung zeigt. Wegen dieser synergistischen Verbesserung einer Vielzahl an anwendungsrelevanten Parametern ist es nach Einschätzung der Erfinder auch besonders vorteilhaft, die vorliegende Erfindung unter Einsatz einer Silikon-Abformmasse als Grundsystem umzusetzen. Bevorzugt ist entsprechend ein erfindungsgemäßes Mehrkomponentensystem, wobei das eine oder die mehreren (Co-)Polymere ausgewählt sind aus der Gruppe bestehend aus funktionalisierten Organopolysiloxanen.

Bevorzugt ist bei den Silikon-Abformmassen beispielsweise ein erfindungsgemäßes Mehrkomponentensystem, wobei das eine oder die mehreren (Co-)Polymere ausgewählt sind aus der Gruppe bestehend aus Allylgruppen umfassenden Organopolysiloxanen Vinylgruppen umfassenden Organopolysiloxanen, bevorzugt mit zwei oder mehr Allylgruppen bzw. Vinylgruppen, besonders bevorzugt endständigen Allylgruppen bzw. Vinylgruppen, im Molekül. Bevorzugt ist dabei zusätzlich oder alternativ ein erfindungsgemäßes Mehrkomponentensystem, wobei das Vernetzersystem eine oder mehrere Vernetzer-Verbindungen umfasst, die ausgewählt sind aus der Gruppe bestehend aus Organohydrogenpolysiloxanen, wobei die Vernetzer-Verbindung und das eine oder die mehreren (Co-)Polymere bevorzugt zumindest teilweise in der gleichen Komponente des Mehrkomponentensystems vorliegen. Bevorzugt ist dabei zusätzlich oder alternativ auch ein erfindungsgemäßes Mehrkomponentensystem, wobei das Vernetzersystem eine oder mehrere Katalysator-Verbindungen umfasst, die ausgewählt sind aus der Gruppe bestehend aus Edelmetall-Katalysatoren, bevorzugt Platin-Katalysatoren.

Als Alternative zu den vorstehenden Silikon-Abformmassen kommen insbesondere Systeme in Frage in denen die Vernetzung über eine Kondensation zwischen OH-Gruppen und mit Alkoxysilan-Gruppen erfolgt. Bevorzugt ist ein erfindungsgemäßes Mehrkomponentensystem, wobei das eine oder die mehreren (Co-)Polymere ausgewählt sind aus der Gruppe bestehend aus Hydroxygruppen umfassenden Organopolysiloxanen. Bevorzugt ist zusätzlich oder alternativ ein erfindungsgemäßes Mehrkomponentensystem, wobei das Vernetzersystem eine oder mehrere Vernetzer-Verbindungen umfasst, die ausgewählt sind aus der Gruppe bestehend aus Kieselsäureestern, bevorzugt Alkoxysilan-Gruppen umfassenden Organopolysiloxanen, wobei die Vernetzer-Verbindung und das eine oder die mehreren (Co-)Polymere bevorzugt nicht in der gleichen Komponente des Mehrkomponentensystems vorliegen. Bevorzugt ist zusätzlich oder alternativ auch ein erfindungsgemäßes Mehrkomponentensystem, wobei das Vernetzersystem eine oder mehrere Katalysator-Verbindungen umfasst, die ausgewählt sind aus der Gruppe bestehend aus Organometallverbindungen, bevorzugt Organozinn-Verbindungen, beispielsweise Dioctylzinnoxid, Dibutylzinnoxid und Komplexen dieser Organozinn-Verbindungen mit Monocarbonsäuren, insbesondere Dilaurate.

Erfindungsgemäß umfasst das Mehrkomponentensystem einen oder mehrere Füllstoffe, wobei zumindest einer dieser Füllstoffe ein plättchenförmiger Füllstoff sein muss, wobei auch zwei oder mehr verschiedene Arten an plättchenförmigen Füllstoff eingesetzte werden können. Qualitativ lassen sich diese plättchenförmigen Füllstoffe auch als "Flakes" bezeichnen. Mit anderen Worten handelt es sich somit um ein erfindungsgemäßes Mehrkomponentensystem, wobei der plättchenförmige Füllstoff die Form von Flakes aufweist.

Die plättchenförmigen Füllstoffe werden im Rahmen der vorliegenden Erfindung durch zwei Formfaktoren charakterisiert, wobei ein Füllstoff, welcher diese Formfaktoren erfüllt, als plättchenförmiger Füllstoff angesehen wird.

Der erste Formfaktor F1 = Dₒᵣₜₕₒ/Dₘₐₓ definiert in anderen Worten das Verhältnis der Plättchenbreite zur Plättchenlänge und drückt in Folge des definierten Bereichs aus, dass es sich bei dem plättchenförmigen Füllstoff nicht um stäbchen- oder nadelförmige Füllstoffe handelt. Bevorzugt ist ein erfindungsgemäßes Mehrkomponentensystem, wobei der plättchenförmige Füllstoff einen ersten Formfaktor F1 = Dₒᵣₜₕₒ/Dₘₐₓ im Bereich von 0,35 bis 1,0, bevorzugt im Bereich von 0,4 bis 1,0, besonders bevorzugt im Bereich von 0,45 bis 1,0, ganz besonders bevorzugt im Bereich von 0,5 bis 1,0, aufweist.

Der zweiten Formfaktor F2 = Dₘₐₓ/H beschreibt das Verhältnis zwischen der Plättchenlänge und der Plättchendicke und drückt aus, dass es sich um vergleichsweise flache Plättchen handelt. Bevorzugt ist ein erfindungsgemäßes Mehrkomponentensystem, wobei der plättchenförmige Füllstoff einen zweiten Formfaktor F2 = Dₘₐₓ/H von 4 oder mehr, bevorzugt 5 oder mehr, besonders bevorzugt 6 oder mehr, aufweist. Bevorzugt ist zusätzlich oder alternativ ein erfindungsgemäßes Mehrkomponentensystem, wobei der plättchenförmige Füllstoff einen zweiten Formfaktor F2 = Dₘₐₓ/H im Bereich von 3 bis 90, bevorzugt im Bereich von 4,5 bis 60, besonders bevorzugt im Bereich von 5 bis 30, aufweist.

Den Erfindern ist es insoweit in eigenen Experimenten gelungen, hinsichtlich der absoluten Größenbereiche besonders geeignete Abmessungen für die plättchenförmigen Füllstoffe zu identifizieren, mit denen sich der Zielkonflikt zwischen den rheologischen Eigenschaften, insbesondere der Viskosität und dem Fließverhalten, der Komponenten bzw. der noch nicht vollständig ausgehärteten Abformmasse auf der einen Seite und den mechanischen Eigenschaften der ausgehärteten dentalen Abformmasse auf der anderen Seite besonders vorteilhaft lösen lässt.

Bevorzugt ist nämlich ein erfindungsgemäßes Mehrkomponentensystem, wobei der plättchenförmige Füllstoff einen maximalen Plättchen-Durchmesser in der Plättchen-Ebene Dₘₐₓ im Bereich von 1 bis 600 µm, bevorzugt im Bereich von 2 bis 500 µm, besonders bevorzugt im Bereich von 3 bis 400 µm, ganz besonders bevorzugt im Bereich von 4 bis 200 µm, insbesondere bevorzugt im Bereich von 5 bis 100 µm, aufweist. Bevorzugt ist zusätzlich oder alternativ ein erfindungsgemäßes Mehrkomponentensystem, wobei der plättchenförmige Füllstoff eine mittlere Plättchen-Dicke orthogonal zur Plättchen-Ebene H im Bereich von 0,2 bis 20 µm, bevorzugt im Bereich von 0,5 bis 15 µm, besonders bevorzugt im Bereich von 0,7 bis 10 µm, ganz besonders bevorzugt im Bereich von 1,0 bis 6,0 µm, aufweist, und/oder wobei der plättchenförmige Füllstoff eine mittlere Plättchen-Dicke orthogonal zur Plättchen-Ebene H im Bereich von 2 bis 10 µm, bevorzugt im Bereich von 2,5 bis 8 µm, besonders bevorzugt im Bereich von 3 bis 6 µm, aufweist.

Mit Blick auf die Größenverteilung der Plättchenlänge bevorzugt ist ein erfindungsgemäßes Mehrkomponentensystem, wobei der plättchenförmige Füllstoff eine volumenbezogenen D50 Wert des maximalen Plättchen-Durchmessers in der Plättchen-Ebene D50ₘₐₓ im Bereich von 5 bis 50 µm, bevorzugt im Bereich von 6 bis 40 µm, besonders bevorzugt im Bereich von 7 bis 30 µm, aufweist und/oder wobei der plättchenförmige Füllstoff eine volumenbezogenen D50 Wert des maximalen Plättchen-Durchmessers in der Plättchen-Ebene D50ₘₐₓ im Bereich von 10 bis 40 µm, bevorzugt im Bereich von 12 bis 35 µm, besonders bevorzugt im Bereich von 14 bis 30 µm, ganz besonders bevorzugt im Bereich von 16 bis 25 µm, aufweist.

Ohne an diese Theorie gebunden sein zu wollen, gehen die Erfinder auf Grundlage der durchgeführten Experimente davon aus, dass es die spezifische Morphologie der plättchenförmigen Füllstoffe ist, die die vorteilhaften Effekte der vorliegenden Erfindung auf die rheologischen Eigenschaften bewirkt. Insoweit kann es als Vorteil der vorliegenden Erfindung gesehen werden, dass diese hinsichtlich der chemischen Natur der einzusetzenden plättchenförmigen Füllstoffe prinzipiell sehr flexibel ist, so dass beispielsweise auch plättchenförmige Kunststoffe eingesetzt werden können, was beispielsweise hinsichtlich des Gesamtgewichts der Mehrkomponentensysteme vorteilhaft sein kann. Nach Einschätzung der Erfinder sind einige der mechanischen Eigenschaften, welche durch die Füllung mit Füllstoff beeinflusst werden, insbesondere die Härte, jedoch stärker von den Materialeigenschaften des Füllstoffs abhängig, wobei es nach Einschätzung der Erfinder prinzipiell vorteilhaft ist, statt zumeist relativ weichen Kunststoffen auf anorganische Materialien zurückzugreifen. Beispielhaft ist ein erfindungsgemäßes Mehrkomponentensystem, wobei der plättchenförmige Füllstoff ein organischer oder anorganischer, bevorzugt ein anorganischer, Füllstoff ist. Beispielhaft ist zusätzlich oder alternativ erfindungsgemäßes Mehrkomponentensystem, wobei der plättchenförmige Füllstoff ein amorpher Füllstoff ist.

Als besonders bevorzugt erachten die Erfinder den Einsatz von Glasflakes, welche in Mehrkomponentensystemen und daraus herstellbaren dentalen Abformmassen mit einem besonders vorteilhaften Eigenschaftsprofil resultieren. Bevorzugt ist entsprechend ein erfindungsgemäßes Mehrkomponentensystem, wobei der plättchenförmige Füllstoff zu einem Massenanteil von 90 % oder mehr, bevorzugt 95 % oder mehr, besonders bevorzugt 99 % oder mehr, ganz besonders bevorzugt im Wesentlichen vollständig, aus einem Glas besteht, wobei das Glas bevorzugt ausgewählt ist aus der Gruppe bestehend aus Silikatgläsern und Boratgläsern, bevorzugt Borosilikatgläsern, besonders bevorzugt Aluminoborosilikatgläsern.

Als ganz besonders bevorzugt erachten es die Erfinder, wenn als plättchenförmiger Füllstoff ein Silicium-haltiger plättchenförmiger Füllstoff eigesetzt wird, beispielsweise ein Silikatglas. Insbesondere in der Kombination mit Silikon-Abformmassen werden hierbei besonders vorteilhafte Verbesserungen im Verhältnis von Endhärte zu Reißdehnung erreicht.

Den Erfindern ist es gelungen, für den Gehalt an plättchenförmigen Füllstoffen besonders geeignete Anteile zu identifizieren, wobei diese sowohl auf die Gesamtmasse des Mehrkomponentensystems als auch auf die Gesamtmasse des eingesetzten Füllstoffsystems bezogen werden können. Bevorzugt ist nämlich ein erfindungsgemäßes Mehrkomponentensystem, wobei das Mehrkomponentensystem den plättchenförmigen Füllstoff in einem kombinierten Massenanteil im Bereich von 0,2 bis 75%, bevorzugt im Bereich von 0,5 bis 50 %, besonders bevorzugt im Bereich von 1 bis 40 %, ganz besonders bevorzugt im Bereich von 2 bis 30 %, insbesondere bevorzugt im Bereich von 3 bis 25 %, überaus bevorzugt im Bereich von 5 bis 20 %, umfasst, bezogen auf die Gesamtmasse des Mehrkomponentensystems. Bevorzugt ist zusätzlich oder alternativ auch ein erfindungsgemäßes Mehrkomponentensystem, wobei das Mehrkomponentensystem den plättchenförmigen Füllstoff in einem kombinierten Massenanteil von 5 % oder mehr, bevorzugt 15 % oder mehr, besonders bevorzugt 25 % oder mehr, ganz besonders bevorzugt 45 % oder mehr, insbesondere bevorzugt 75 % oder mehr, potentiell bevorzugt von 95 % oder mehr, umfasst, bezogen auf die Gesamtmasse an Füllstoffen im Mehrkomponentensystem.

Neben dem oder den plättchenförmigen Füllstoffen können in den erfindungsgemäßen Mehrkomponentensystemen auch weitere Füllstoffe vorgesehen werden, wobei insbesondere auf solche üblichen Füllstoffe zurückgegriffen werden kann, welche auch im Stand der Technik für dentale Abformmassen zum Einsatz kommen. Bevorzugt ist ein erfindungsgemäßes Mehrkomponentensystem, wobei das Mehrkomponentensystem neben dem zumindest einen plättchenförmigen Füllstoff zumindest einen weiteren Füllstoff umfasst, bei dem es sich nicht um einen plättchenförmigen Füllstoff handelt, wobei der weitere Füllstoff bevorzugt ausgewählt ist aus der Gruppe bestehend aus Gesteinsmehlen, Silikaten, Carbonaten, Sulfaten, Quarzmehlen, Cristobalitmehlen, gefälltem amorphen Siliciumdioxid oder pyrogenem amorphen Siliciumdioxid, Stärke, besonders bevorzugt bestehend aus Quarzmehlen, Cristobalitmehlen, gefälltem amorphen Siliciumdioxid oder pyrogenem amorphen Siliciumdioxid.

Bevorzugt ist ein erfindungsgemäßes Mehrkomponentensystem, wobei das Mehrkomponentensystem einen Gesamtfüllstoffgehalt von 40 % oder mehr, bevorzugt von 45 % oder mehr, besonders bevorzugt von 50 % oder mehr, ganz besonders bevorzugt von 55 % oder mehr, aufweist, bezogen auf die Gesamtmasse des Mehrkomponentensystems. Bevorzugt ist zusätzlich oder alternativ ein erfindungsgemäßes Mehrkomponentensystem, wobei der kombinierte Massenanteil sämtlicher Füllstoffe in dem Mehrkomponentensystem im Bereich von 20 bis 80 %, bevorzugt im Bereich von 30 bis 75 %, besonders bevorzugt im Bereich von 40 bis 70 %, liegt, bezogen auf die Gesamtmasse des Mehrkomponentensystems.

Den Erfindern ist es gelungen, besonders vorteilhafte Massenverhältnisse zwischen den plättchenförmigen und den weiteren Füllstoffen zu identifizieren. Bevorzugt ist ein erfindungsgemäßes Mehrkomponentensystem, wobei der Quotient aus dem kombinierten Massenanteil der plättchenförmigen Füllstoffe geteilt durch den kombinierten Massenanteil der weiteren Füllstoffe im Bereich von 0,01 bis 1,0, bevorzugt im Bereich von 0,015 bis 0,7, besonders bevorzugt im Bereich von 0,02 bis 0,6, ganz besonders bevorzugt im Bereich von 0,025 bis 0,5, liegt.

Die Erfinder haben gefunden, dass sich der Einsatz des plättchenförmigen Füllstoffs vorteilhaft auf die rheologischen Eigenschaften der Komponenten des Mehrkomponentensystems auswirkt, unabhängig davon, welche Bestandteile des Vernetzungssystems beispielsweise ebenfalls in dieser Komponente vorgelegt werden. Entsprechend erachten es die Erfinder für besonders vorteilhaft, den plättchenförmigen Füllstoff in mehreren, bevorzugt sämtlichen, Komponenten des Mehrkomponentensystems einzusetzen. Bevorzugt ist entsprechend ein erfindungsgemäßes Mehrkomponentensystem, wobei das Mehrkomponentensystem den zumindest einen plättchenförmigen Füllstoff in zwei oder mehr, bevorzugt in drei oder mehr, besonders bevorzugt in sämtlichen separaten Komponenten umfasst. Besonders bevorzugt ist ein erfindungsgemäßes Mehrkomponentensystem, wobei der plättchenförmige Füllstoff in zwei oder mehr der separaten Komponenten des Mehrkomponentensystems vorliegt, bevorzugt in sämtlichen separaten Komponenten des Mehrkomponentensystems vorliegt, wobei sich der Massenanteil des plättchenförmigen Füllstoffs bezogen auf die Masse der Komponente in den separaten Komponenten bevorzugt um 80 % oder weniger, bevorzugt 60 % oder weniger unterscheidet, wobei sich der Massenanteil des plättchenförmige Füllstoffs bezogen auf die Gesamtmasse an Füllstoff in der jeweiligen Komponente bevorzugt um 20 % oder weniger, bevorzugt um 10 % oder weniger, besonders bevorzugt um 5 % oder weniger, unterscheidet.

Eine besonders vorteilhafte Einstellung der rheologischen Eigenschaften bei der Verarbeitung und der mechanischen Eigenschaften der gehärteten Abformmasse lässt sich nach Erkenntnissen der Erfinder insbesondere auch durch die Kombination von zwei oder mehr verschiedenen plättchenförmigen Füllstoffen realisieren, welche bevorzugt in der gleichen Komponente, besonders bevorzugt jeweils in sämtlichen Komponenten, eingesetzt werden können. Bevorzugt ist demgemäß ein erfindungsgemäßes Mehrkomponentensystem, wobei das Mehrkomponentensystem zwei oder mehr plättchenförmige Füllstoffe umfasst, bevorzugt genau zwei, besonders bevorzugt in einem Verhältnis der Massenanteile im Bereich von 4:1 bis 1:4, ganz besonders bevorzugt im Bereich von 2:1 bis 1:2, insbesondere bevorzugt im Bereich von 1,5:1 bis 1:1,5, wobei sich die zwei oder mehr plättchenförmige Füllstoffe bevorzugt hinsichtlich zumindest eines Aspektes unterscheiden, der ausgewählt ist aus der Gruppe bestehend aus dem Formfaktor F1, dem Formfaktor F2, dem maximalen Plättchen-Durchmesser Dₘₐₓ, der mittleren Plättchen-Dicke H und der chemischen Zusammensetzung, besonders bevorzugt hinsichtlich der chemischen Zusammensetzung und zumindest eines weiteren Aspektes unterscheiden, der ausgewählt ist aus der Gruppe bestehend aus dem Formfaktor F1, dem Formfaktor F2, dem maximalen Plättchen-Durchmesser Dₘₐₓ und der mittleren Plättchen-Dicke H.

Hinsichtlich der Verteilung der Bestandteile auf die Komponenten des Mehrkomponentensystems bevorzugt ist beispielsweise ein erfindungsgemäßes Mehrkomponentensystem, wobei das Mehrkomponentensystem eine erste Komponente A umfasst, wobei die Komponente A enthält:
A.i) ein oder mehrere vernetzbare (Co-)Polymere,
A.ii.a) eine oder mehrere Vernetzer-Verbindungen, und
A.iv) einen oder mehrere Füllstoffe, wobei die Komponente A als Füllstoff bevorzugt zumindest einen plättchenförmigen Füllstoff umfasst.

Bevorzugt ist insoweit zusätzlich oder alternativ, bevorzugt zusätzlich, zudem ein erfindungsgemäßes Mehrkomponentensystem, wobei das Mehrkomponentensystem eine zweite Komponente B umfasst, wobei die Komponente B enthält:
B.i) ein oder mehrere vernetzbare (Co-)Polymere,
B.ii.b) eine oder mehrere Katalysator-Verbindungen und/oder Aktivator-Verbindungen und/oder Initiator-Verbindungen, bevorzugt Katalysator-Verbindungen,
und optional aber bevorzugt:
   B.iii) einen oder mehrere Füllstoffe, wobei die Komponente B als Füllstoff bevorzugt zumindest einen plättchenförmigen Füllstoff umfasst.

Hinsichtlich der Verteilung der Bestandteile auf die Komponenten des Mehrkomponentensystems bevorzugt ist alternativ beispielsweise auch ein erfindungsgemäßes Mehrkomponentensystem, wobei das Mehrkomponentensystem eine erste Komponente A umfasst, wobei die Komponente A enthält:
A.i) ein oder mehrere vernetzbare (Co-)Polymere, und
A.iv) einen oder mehrere Füllstoffe, wobei die Komponente A als Füllstoff bevorzugt zumindest einen plättchenförmigen Füllstoff umfasst.

Bevorzugt ist insoweit zusätzlich oder alternativ, bevorzugt zusätzlich, zudem ein erfindungsgemäßes Mehrkomponentensystem, wobei das Mehrkomponentensystem eine zweite Komponente B umfasst, wobei die Komponente B enthält:
B.ii.c) eine oder mehrere Aktivatorverbindungen-Verbindungen,
und optional aber bevorzugt:
   B.iii) einen oder mehrere Füllstoffe, wobei die Komponente B als Füllstoff bevorzugt zumindest einen plättchenförmigen Füllstoff umfasst.

Es kann als Vorteil der erfindungsgemäßen Mehrkomponentensysteme gesehen werden, dass dieses hinsichtlich der Anwesenheit weiterer typischer Additive sehr flexibel ist und sich entsprechend durch den Einsatz entsprechender Additive gezielt auf bestimmte Anwendungserfordernisse abstimmen lässt. Bevorzugt ist für viele Anwendungen somit ein erfindungsgemäßes Mehrkomponentensystem, wobei das Mehrkomponentensystem zusätzlich umfasst:
iv) ein oder mehrere weitere Additive, wobei die weiteren Additive ausgewählt sind aus der Gruppe bestehend aus Thixotropiermitteln, Pigmenten, Farbstoffen, Hydrophilierungsmitteln, Stabilisatoren, Inhibitoren, Geruchsstoffen und Geschmacksstoffen.

Die Erfindung betrifft auch eine dentale Abformmasse, hergestellt oder herstellbar durch Vermischen der separaten Komponenten des erfindungsgemäßen Mehrkomponentensystems. Offenbart wird in diesem Zusammenhang zudem die Verwendung eines erfindungsgemäßen Mehrkomponentensystems zur Herstellung einer dentalen Abformmasse durch Mischen der separaten Komponenten des Mehrkomponentensystems.

Die Erfindung betrifft zudem die Verwendung eines plättchenförmigen Füllstoffs als Füllstoff in Mehrkomponentensystemen zur Herstellung einer dentalen Abformmassen zur Verbesserung der rheologischen Eigenschaften der Komponenten des Mehrkomponentensystems bzw. der Abformmasse,
wobei der plättchenförmige Füllstoff einen ersten Formfaktor F1 = Dₒᵣₜₕₒ/Dₘₐₓ im Bereich von 0,3 bis 1,0 und einen zweiten Formfaktor F2 = Dₘₐₓ/H von 3 oder mehr aufweist,
wobei Dₘₐₓ der maximale Plättchen-Durchmesser in der Plättchen-Ebene ist, wobei Dₒᵣₜₕₒ der größte Plättchen-Durchmesser orthogonal zu Dₘₐₓ in der Plättchen-Ebene ist, und wobei H die mittlere Plättchen-Dicke orthogonal zur Plättchen-Ebene ist.

Nachfolgend werden die Erfindung und bevorzugte Ausführungsformen der Erfindung unter Bezugnahme auf die beiliegende Figur näher erläutert und beschrieben. Dabei zeigt:

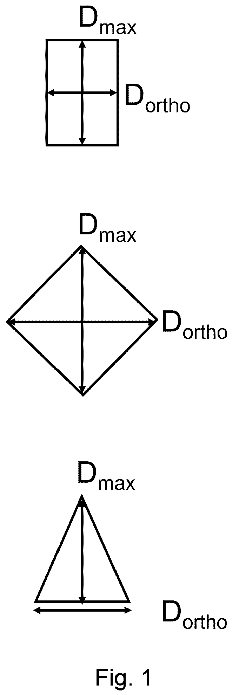

- Fig. 1: eine schematische Visualisierung der zur Berechnung der Formfaktoren der plättchenförmigen Füllstoffe herangezogenen Bezugsgrößen.

Fig. 1 zeigt in einer schematischen Visualisierung an drei beispielhaften, abstrahierten Formen von plättchenförmigen Füllstoffen in der Draufsicht auf die Plättchen-Ebene, wie der maximale Plättchen-Durchmesser Dₘₐₓ und der größte hierzu orthogonal stehende Plättchen-Durchmesser Dₒᵣₜₕₒ bestimmt werden.

Nachfolgend werden die Erfindung und bevorzugte Ausführungsformen der Erfindung zudem unter Bezugnahme auf Experimente näher erläutert und beschrieben.

### A. Untersuchungen:

Im Rahmen der Experimente wurden die folgenden Versuche durchgeführt.

An den einzelnen Komponenten (Basispate BP und Katalysatorpaste KP) der Proben wurden die rheologischen Eigenschaften bestimmt. Die Messungen der der rheologischen Eigenschaften wurden an einem Rheometer der Fa. Thermo-Fischer durchgeführt. Zur Anwendung kam die Messgeometrie Platte/Kegel. Für diese Messungen rotiert der Kegel auf einer Substanz, die sich auf der stationären Platte befindet. Hierbei ist der Messspalt zwischen Kegel und Platte, sowie die Temperatur (23 °C) über die gesamte Messung konstant. Die Viskosität beschreibt den Widerstand einer Flüssigkeit gegenüber einer Scherkraft und ist somit als Zähigkeit bekannt. Die Fließgrenze beschreibt eine Kraft, die benötigt wird, um ein Stoff zum Fließen zu bringen.

Zur Messung der Viskosität und der Fließgrenze wird die Scherrate des Kegels von 0,00 1/s bis 5,00 1/s in einem Zeitraum von 3,00 min erhöht. Die Viskosität in Pa*s wird bei einer Scherrate von 3,00 1/s gemessen. Die Fließgrenze in Pa ergibt sich aus Interpolation / Linearer Regression der Datenpunkte bei rücklaufender Scherrate und ist als Achsenabschnitt "a" (ergibt sich aus y=a+bx) genkennzeichnet.

An den Mischungen der Basispate BP und der Katalysatorpaste KP wurde der Mischdiskus nach DIN ISO 4823 2021-06 mit einer Apparatur gemäß A.1 (zwei quadratische Glasplatten ca. 60 x 60 mm, Mindestabstand 3 mm, Probenmenge 0,5 +/- 0,02 ml, Belastungszeit 5 Min, Messtemperatur: 23 °C +/- 2 °C) durchgeführt.

An den dentalen Abformmassen wurde die Shore A Härte nach 10 min, 1 h und 24 h bestimmt.

Zudem wurden für die dentalen Abformmassen die Reißdehnung gemäß DIN 53504:2017-03 und der E-Modul (Biege) gemäß DIN13903:2005-10 bestimmt.

Darüber hinaus wurde an den dentalen Abformmassen der etablierte "Shark-Fin-Test" (M. Zenginel et al., Deutsche Zahnärztliche Zeitschrift, 2011; 66 (12), "Shark Fin Test und rheologische Eigenschaften elastomerer Abformmaterialien: Eine Korrelationsanalyse"; DOI 10.3238/dzz.2011.0899) durchgeführt, welcher die Fließeigenschaften der Abformmaterialien im gemischten Zustand visuell, in Form einer Flossenhöhe anzeigt.

### A. Probenherstellung - Versuchsreihe 1:

In den Experimenten der ersten Versuchsreihe kamen vier verschiedene Grundrezepturen GZ1 bis GZ4 auf der Basis von Polydimethylsiloxan (Vinylterminiert, CAS: 68083-19-2, Gesamtmassenanteil im Bereich von ca. 20 bis 30 %) und dem Vernetzer Methylhydrogenpolysiloxan (CAS: 68037-59-2; Gesamtmassenanteil im Bereich von ca. 5 bis 8 %) zum Einsatz, welche sich hinsichtlich der eingesetzten Additive und der Gesamtmassenanteile der Komponenten, insbesondere im Gesamtfüllstoffgehalt, sowie deren Verteilung auf die sogenannte Basispaste (BP) und eine sogenannte Katalysatorpaste (KP) unterscheiden.

Bei den Grundrezepturen GZ1 bis GZ4 handelt es sich um geeignete Modellsysteme, an welchen sich der Einfluss der Füllstoffe nach Einschätzung der Erfinder ausgezeichnet nachvollziehen lässt, wobei die Grundrezepturen GZ1 bis GZ4 einen guten Eindruck über kommerziell relevante Produkte erlauben. Ausgehend von diesen Modellsystemen wird bei gleichem Gesamtfüllstoffgehalt jeweils nur die Zusammensetzung der Füllstoffe verändert. Vor diesem Hintergrund kann vorliegend aus Gründen der Übersichtlichkeit vorteilhafterweise von einer umfassenderen Auflistung der Bestandteile der Grundrezepturen abgesehen werden.

Der relevante Gesamtfüllstoffanteile der Grundrezepturen GZ1 bis GZ4 ist in der nachfolgenden Tabelle 1 aufgeführt.

**Tabelle 1 - Gesamtfüllstoffanteil der Grundrezepturen GZ1 bis GZ4 bezogen auf die Gesamtmasse der Grundrezepturen**

| Grundrezeptur | Gesamtfüllstoffanteil /% |
|---|---|
| GZ1 | 44,01 |
| GZ2 | 44,30 |
| GZ3 | 49,42 |
| GZ4 | 49,46 |

Ausgehend von den Grundrezepturen GZ1 bis GZ4 wurden die Proben hergestellt. Hierbei kamen die in Tabelle 2 zusammengefassten Füllstoffe zum Einsatz.

**Tabelle 2 - Eingesetzte Füllstoffe**

| Abkürzung | Anmerkung |
|---|---|
| Füllstoff 1 | Plättchenförmiger Füllstoff aus Alumnioborosilikatglas, D50ₘₐₓ = 8 - 12 µm, H = 1,0 - 1,3 µm, Handelsname GF001-10, Firma: Glassflake Limited |
| Füllstoff 2 | Plättchenförmiger Füllstoff aus Alumnioborosilikatglas, D50ₘₐₓ = 18 - 22 µm, H = 3,5 - 5,5 µm, Handelsname GF005-20, Firma: Glassflake Limited |
| Füllstoff 3 | Füllstoff auf Quarzbasis (Cristobalit), D50ₘₐₓ = 2,5 µm; Handelsname Silbond |
| Füllstoff 4 | Füllstoff auf Basis von amorphem Siliciumdioxid (CAS: 7631-86-9) |

Die hergestellten Proben sind in der Tabelle 3 zusammengefasst, wobei jeweils dargestellt ist, in welchem Gesamtmassenanteil die jeweiligen Füllstoffe eingesetzte werden.

**Tabelle 3 - Füllstoffanteile der Proben, alle Angaben in Massenanteilen in %, bezogen auf die Gesamtmasse der Grundrezepturen - Versuchsreihe 1**

| Probe | Grundrezeptur | Füllstoff 1 | Füllstoff 2 | Füllstoff 3 | Füllstoff 4 |
|---|---|---|---|---|---|
| V1 | GZ1 | 0 | 0 | 37,56 | 6,45 |
| E1 | GZ1 | 0 | 5 | 32,56 | 6,45 |
| E2 | GZ1 | 0 | 10 | 27,56 | 6,45 |
| V2 | GZ2 | 0 | 0 | 37,85 | 6,45 |
| E3 | GZ2 | 0 | 10 | 27,85 | 6,45 |
| E4 | GZ2 | 10 | 0 | 27,85 | 6,45 |
| E5 | GZ2 | 0 | 10 | 27,85 | 6,45 |
| E6 | GZ2 | 0 | 10 | 27,85 | 6,45 |
| V3 | GZ3 | 0 | 0 | 43,55 | 5,87 |
| E7 | GZ3 | 0 | 18,23 | 25,32 | 5,87 |
| E8 | GZ3 | 18,23 | 0 | 25,32 | 5,87 |
| E9 | GZ3 | 9,11 | 9,11 | 25,32 | 5,87 |
| E10 | GZ3 | 4,55 | 13,66 | 25,34 | 5,87 |
| E11 | GZ3 | 13,66 | 4,55 | 25,34 | 5,87 |
| V4 | GZ4 | 0 | 0 | 43,59 | 5,87 |
| E12 | GZ4 | 18,21 | 0 | 25,38 | 5,87 |
| E13 | GZ4 | 28,21 | 0 | 15,38 | 5,87 |
| E14 | GZ4 | 21,78 | 21,78 | 0 | 5,87 |
| E15 | GZ1 | 0 | 10 + 10 | 27,56 | 6,45 |

Mit Ausnahme der Proben E5 und E6 wurden die Füllstoffe 1 und 2 jeweils einer schon vorgemischten Grundrezeptur zugesetzt, welche bereits den Füllstoff 3 enthielt. Um auszuschließen, dass die Herstellungsreihenfolge einen entscheidenden Einfluss auf die Materialeigenschaften hat, wurde in den Proben E5 und E6 der Füllstoff 2 jeweils auch schon bei der Herstellung der restlichen Grundrezeptur zugesetzt und vollständig mit den sonstigen Komponenten verarbeitet.

Die Probe E15 entspricht der Probe E2, wobei zusätzlich weitere 10 % an Füllstoff 2 zugesetzt wurden, so dass ein höherer Gesamtfüllstoffgehalt erhalten wird.

In den Grundrezepturen verteilte sich die Füllstoffmenge auf die Basispaste (BP) und die Katalysatorpaste (KP), wobei etwa 42 bis 52 % des Gesamtfüllstoffs in der Basispaste und etwa 51 bis 65 % des Gesamtfüllstoffs in der Katalysatorpaste eingesetzt wurde. Sofern in den Proben Füllstoff 1 und/oder Füllstoff 2 eingesetzt wurde, wurde dieser zu gleichen Teilen auf die Basispaste und die Katalysatorpaste aufgeteilt, ohne die relativen Füllstoffanteile in den Pasten zu verändern (Probe E2 enthält bezogen auf die Gesamtmasse der Rezeptur beispielsweise jeweils 5 % des Füllstoffs 2 in KP und der BP). Dieses Vorgehen wurde gewählt, da sich hierdurch eine breitere Verteilung der relativen Anteile am Füllstoffsystem untersucht werden können.

Die an den Proben bestimmten Eigenschaften sind in den Tabellen 4 bis 7 zusammengestellt.

**Tabelle 4 - Messwerte - Versuchsreihe 1 - Teil 1**

| Größe | V1 | E1 | E2 |
|---|---|---|---|
| Viskosität BP / (Pa*s) | 147,7 | 120,0 | 115,1 |
| Fließgrenze BP / Pa | 243,7 | 181,7 | 167,8 |
| Viskosität KP / (Pa*s) | 121,0 | 94,54 | 79,96 |
| Fließgrenze KP / Pa | 184,8 | 136,5 | 100,2 |
| Mischdiskus / mm | 37 | 38 | 38 |
| Shore A (10 min) | 49,8 | 48,2 | 48,3 |
| Shore A (1 h) | 50,0 | 48,8 | 48,7 |
| Shore A (24 h) | 50,5 | 48,9 | 49,3 |
| Reißdehung /% | 188 | 201 | 214 |
| E-Modul (Biege) / MPa | 0,58 | 0,54 | 0,56 |
| Shark Fin Test | 6,5 | 8 | 9 |

**Tabelle 5 - Messwerte - Versuchsreihe 1 - Teil 2**

| Größe | V2 | E3 | E4 | E5 | E6 |
|---|---|---|---|---|---|
| Viskosität BP / (Pa*s) | 86,61 | 77,77 | 78,89 | 79,07 | 84,84 |
| Fließgrenze BP / Pa | 132,0 | 107,8 | 110,2 | 112,8 | 123,9 |
| Viskosität KP / (Pa*s) | 126,7 | 81,88 | 84,93 | 89,33 | 94,77 |
| Fließgrenze KP / Pa | 182,3 | 84,71 | 95,04 | 115,6 | 129,3 |
| Mischdiskus /mm | 31,5 | 39 | 38 | 38,5 | 38 |
| Shore A (10 min) | 48,0 | 47,7 | 48,8 | 48,6 | 49,6 |
| Shore A (1 h) | 48,0 | 48,0 | 48,7 | 49,1 | 49,8 |
| Shore A (24 h) | 48,4 | 48,2 | 50,3 | 49,4 | 50,0 |
| Reißdehung /% | 188 | 198 | 169 | 172 | 176 |
| E-Modul (Biege) / MPa | 0,47 | 0,44 | 0,56 | 0,48 | 0,51 |
| Shark Fin Test | 6,9 | 8,2 | 7,5 | 7,5 | 7,17 |

**Tabelle 6 - Messwerte - Versuchsreihe 1 - Teil 3**

| Größe | V3 | E7 | E8 | E9 | E10 | E11 |
|---|---|---|---|---|---|---|
| Viskosität BP / (Pa*s) | 125,9 | 87,08 | 104 | 93,43 | 92,13 | 100,3 |
| Fließgrenze BP / Pa | 213,7 | 125,1 | 155,1 | 134,8 | 134,8 | 145,7 |
| Viskosität KP / (Pa*s) | 257,5 | 106,6 | 131,7 | 112 | 111,5 | 116,1 |
| Fließgrenze KP / Pa | 321,5 | 125,2 | 164,6 | 129,4 | 125,5 | 136,1 |
| Mischdiskus / mm | 34,5 | 37 | 36 | 37 | 37 | 36 |
| Shore A (10 min) | 50,1 | 51,2 | 53,1 | 52,2 | 51,7 | 53,7 |
| Shore A (1 h) | 50,2 | 51,6 | 53,3 | 52,2 | 52,0 | 54,1 |
| Shore A (24 h) | 51,6 | 51,7 | 54,4 | 52,9 | 52,8 | 54,4 |
| Reißdehung /% | 213 | 184 | 169 | 178 | 173 | 152 |
| E-Modul (Biege) / MPa | 0,35 | 0,66 | 0,71 | 0,69 | 0,59 | 0,68 |
| Shark Fin Test | 3,5 | 6 | 5,4 | 6 | 6,2 | 5,5 |

**Tabelle 7 - Messwerte - Versuchsreihe 1 - Teil 4 (n.b. = nicht bestimmt)**

| Größe | V4 | E12 | E13 | E14 | E15 |
|---|---|---|---|---|---|
| Viskosität BP / (Pa*s) | 128,6 | 99,56 | 93,29 | 97,69 | 148,6 |
| Fließgrenze BP / Pa | 218,7 | 146,7 | 125,7 | 132,7 | 218,0 |
| Viskosität KP / (Pa*s) | 241,4 | 120,0 | 101,5 | 86,61 | 92,8 |
| Fließgrenze KP / Pa | 268,6 | 122,1 | 81,6 | 46,55 | 106,3 |
| Mischdiskus / mm | 35 | 37,5 | 39 | 37 | n.b. |
| Shore A (10 min) | 49,8 | 52 | 53,2 | 50,2 | n.b. |
| Shore A (1 h) | 50,3 | 52,1 | 53 | 50,3 | n.b. |
| Shore A (24 h) | 50,9 | 53,9 | 53,6 | 52,4 | n.b. |
| Reißdehung / % | 211 | 164 | 171 | 238 | n.b. |
| E-Modul (Biege) / MPa | 0,27 | 0,67 | 0,56 | 0,55 | n.b. |
| Shark Fin Test | 4,06 | 6,21 | 6,29 | 7,07 | 7,5 |

Der erfindungsgemäß vorgesehene Einsatz von plättchenförmigen Füllstoffen (Füllstoffe 1 und 2) führt überraschend zu durchgehend verbesserten rheologischen Eigenschaften, in Bezug auf die Fließfähigkeit der Materialien, und - resultierend daraus - zu erhöhten Mischdisken, im Vergleich mit üblichen Füllstoffen für Abformmaterialien, wobei der vorteilhafte Effekt mit steigendem Anteil des plättchenförmigen Füllstoffs am Gesamtfüllstoffgehalt besonders ausgeprägt ist.

In Übereinstimmung mit diesem Befund werden vorteilhafte Werte für den Mischdiskus und eine signifikante Verbesserung im Shark Fin Test erzielt. Vorteilhafterweise verbleiben die mechanischen Eigenschaften dabei zumindest auf einem vergleichbaren Niveau oder werden sogar verbessert. Insbesondere zeigt sich in vielen Fällen ein günstiges Eigenschaftsprofil im Verhältnis der regelmäßig konkurrierende Werte zwischen Härte und Reißdehnung.

Der Vergleich von V1, E1, E2 und E15 verdeutlicht, dass sich durch den erfindungsgemäß vorgesehenen Einsatz von plättchenförmigen Füllstoffen bei vergleichbaren bzw. sogar leicht verbesserten Fließfähigkeiten höhere Gesamtfüllstoffgehalte realisieren lassen.

### B. Probenherstellung - Versuchsreihe 2:

In den Experimenten der zweiten Versuchsreihe wurden weitere Proben ausgehend von einer Basispaste-Grundrezeptur BGZ mit einem Ausgangsfüllstoffgehalt eines Füllstoffs auf Quarzbasis von 33 % erzeugt. Hierbei wurden der Basispaste-Grundrezeptur zusätzlicher Füllstoff zugegeben. Die entsprechenden Zusammensetzungen sind in Tabelle 8 zusammengefasst.

**Tabelle 8 - Füllstoffanteile der Proben, alle Angaben in Massenanteilen in %, bezogen auf die Gesamtmasse der Basispaste-Grundrezepturen - Versuchsreihe 2**

| Probe | Füllstoff 1 | Füllstoff 2 | Füllstoff 3 |
|---|---|---|---|
| V5 | 0 | 0 | 34 |
| V6 | 0 | 0 | 35,5 |
| V7 | 0 | 0 | 38 |
| E16 | 1 | 0 | 33 |
| E17 | 2,5 | 0 | 33 |
| E18 | 5 | 0 | 33 |
| E19 | 0 | 1 | 33 |
| E20 | 0 | 2,5 | 33 |
| E21 | 0 | 5 | 33 |

Die an den Proben erhaltenen Messwerte sind in Tabelle 9 zusammengefasst.

**Tabelle 9 - Messwerte - Versuchsreihe 2**

| Probe | Viskosität BP / (Pa*s) | Fließgrenze BP / Pa |
|---|---|---|
| V5 | 141,1 | 225,3 |
| V6 | 150,4 | 242,9 |
| V7 | 166,0 | 271,7 |
| E16 | 139,1 | 223,5 |
| E17 | 143,4 | 229,0 |
| E18 | 158,4 | 254,0 |
| E19 | 131,9 | 212,6 |
| E20 | 137,6 | 219,4 |
| E21 | 144,9 | 231,9 |

Auch diese Werte belegen, dass der erfindungsgemäß vorgesehene Einsatz von plättchenförmigen Füllstoffen zu durchgehend verbesserten Fließfähigkeiten bzw. Mischdisken, im Vergleich mit üblichen Füllstoffen für Abformmaterialien führt, wobei der vorteilhafte Effekt mit steigendem Anteil des plättchenförmigen Füllstoffs am Gesamtfüllstoffgehalt besonders ausgeprägt sind, wobei wiederum deutlich wird, dass sich bei vergleichbaren rheologischen Eigenschaften höhere Gesamtfüllstoffgehalte realisieren lassen.

## Patentansprüche

1. Mehrkomponentensystem zur Herstellung einer dentalen Abformasse, umfassend in zwei oder mehr separaten Komponenten:
i) ein oder mehrere vernetzbare (Co-)Polymere,
ii) einen oder mehrere Bestandteile eines Vernetzersystems zur Vernetzung der vernetzbaren (Co-)Polymere, und
iii) einen oder mehrere Füllstoffe,
wobei das Mehrkomponentensystem zumindest einen plättchenförmigen Füllstoff umfasst,
wobei der plättchenförmige Füllstoff einen ersten Formfaktor F1 = Dₒᵣₜₕₒ/Dₘₐₓ im Bereich von 0,3 bis 1,0 und einen zweiten Formfaktor F2 = Dₘₐₓ/H von 3 oder mehr aufweist,
wobei Dₘₐₓ der maximale Plättchen-Durchmesser in der Plättchen-Ebene ist, wobei Dₒᵣₜₕₒ der größte Plättchen-Durchmesser orthogonal zu Dₘₐₓ in der Plättchen-Ebene ist, und wobei H die mittlere Plättchen-Dicke orthogonal zur Plättchen-Ebene ist.

2. Mehrkomponentensystem nach Anspruch 1, wobei das Mehrkomponentensystem den plättchenförmigen Füllstoff in einem kombinierten Massenanteil im Bereich von 0,2 bis 75 % umfasst, bezogen auf die Gesamtmasse des Mehrkomponentensystems.

3. Mehrkomponentensystem nach einem der Ansprüche 1 oder 2, wobei das Mehrkomponentensystem den plättchenförmigen Füllstoff in einem kombinierten Massenanteil von 5 % oder mehr umfasst, bezogen auf die Gesamtmasse an Füllstoffen im Mehrkomponentensystem.

4. Mehrkomponentensystem nach einem der Ansprüche 1 bis 3, wobei der plättchenförmige Füllstoff ein amorpher Füllstoff ist.

5. Mehrkomponentensystem nach einem der Ansprüche 1 bis 4, wobei der plättchenförmige Füllstoff zu einem Massenanteil von 90 % oder mehr aus einem Glas besteht.

6. Mehrkomponentensystem nach einem der Ansprüche 1 bis 5, wobei der plättchenförmige Füllstoff eine volumenbezogenen D50 Wert des maximalen Plättchen-Durchmessers in der Plättchen-Ebene D50ₘₐₓ im Bereich von 5 bis 50 µm aufweist.

7. Mehrkomponentensystem nach einem der Ansprüche 1 bis 6, wobei der plättchenförmige Füllstoff einen ersten Formfaktor F1 = Dₒᵣₜₕₒ/Dₘₐₓ im Bereich von 0,35 bis 1,0 aufweist und/oder wobei der plättchenförmige Füllstoff einen zweiten Formfaktor F2 = Dₘₐₓ/H von 4 oder mehr aufweist.

8. Mehrkomponentensystem nach einem der Ansprüche 1 bis 7, wobei das eine oder die mehreren (Co-)Polymere ausgewählt sind aus der Gruppe bestehend aus funktionalisierten Organopolysiloxanen.

9. Dentale Abformmasse, hergestellt oder herstellbar durch Vermischen der separaten Komponenten des Mehrkomponentensystems nach einem der Ansprüche 1 bis 8.

10. Verwendung eines plättchenförmigen Füllstoffs als Füllstoff in dentalen Abformmassen zur Verbesserung der rheologischen Eigenschaften der Abformmasse, wobei der plättchenförmige Füllstoff einen ersten Formfaktor F1 = Dₒᵣₜₕₒ/Dₘₐₓ im Bereich von 0,3 bis 1,0 und einen zweiten Formfaktor F2 = Dₘₐₓ/H von 3 oder mehr aufweist, wobei Dₘₐₓ der maximale Plättchen-Durchmesser in der Plättchen-Ebene ist, wobei Dₒᵣₜₕₒ der größte Plättchen-Durchmesser orthogonal zu Dₘₐₓ in der Plättchen-Ebene ist, und wobei H die mittlere Plättchen-Dicke orthogonal zur Plättchen-Ebene ist.
